# EUROPEAN PATENT APPLICATION

(11) **EP 4 071 141 A1**
(43) Date of publication of application: **12.10.2022**
(21) Application number: 20896516.0
(22) Date of filing: 27.11.2020
(51) Int. Cl.: C07D 305/14, A61K 9/127, A61P 35/00

(54) **WEAK ALKALINE CABAZITAXEL DERIVATIVE AND FORMULATION THEREOF**

(30) Priority: 03.12.2019 CN 201911218994
(71) Applicant: Shenyang Pharmaceutical University, Shenyang, Liaoning 110016 (CN)
(72) Inventor: WANG, Yong Jun, Shenyang, Liaoning 110016 (CN); YANG, Zi Meng, Shenyang, Liaoning 110016 (CN); HE, Zhong Gui, Shenyang, Liaoning 110016 (CN); LIU, Hong Zhuo, Shenyang, Liaoning 110016 (CN); CHI, Dong Xu, Shenyang, Liaoning 110016 (CN)
(74) Representative: Westphal, Petra
(86) International application number: PCT/CN2020/132491
(87) International publication number: WO 2021/109944

(57) **Abstract**

The present invention relates to a weak alkaline carbazitaxel derivative and a formulation thereof, and specifically relates to the synthesis of the weak alkaline carbazitaxel derivative, a liposome formulation comprising the derivative, and use of the derivative in a drug delivery system, belonging to the technical field of medical technologies. The weak alkaline carbazitaxel derivative connects carbazitaxel to a weak alkaline intermediate by means of an ester bond, and the ester bond can be broken by means of the action of esterase in vivo, so as to release an active drug. The structural formula thereof is as follows: wherein a linker is a C₁-C₄ alkyl, C₃-C₆ cycloalkyl or phenyl; [N] is N-methylpiperazinyl, piperidinyl, 4-(1-piperidinyl)piperidinyl, morpholinyl, tetrahydropyrrolyl, or other tertiary amine structure. The weak alkaline cabazitaxel derivative can be used to prepare a liposome formulation. The liposome formulation has the characteristics of high drug loading, high encapsulation efficiency, and good stability. After the administration by injection, the liposome formulation can greatly improve the circulation time of the drug in vivo, increase the accumulation amount of the drug in the tumor site, and improve the anti-tumor effect and tolerance dosage.

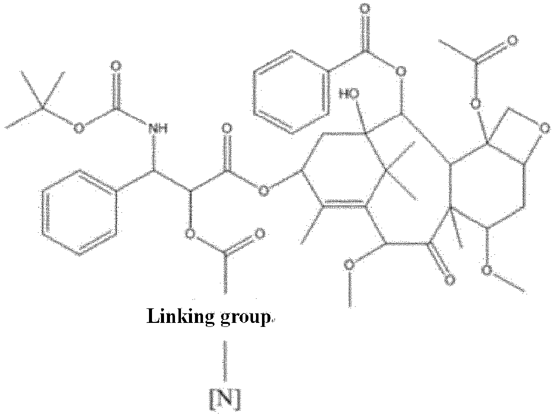

## Description

### TECHNICAL FIELD

The present invention relates to a cabazitaxel weakly-alkaline derivative and a preparation thereof, in particular relates to synthesis of the cabazitaxel weakly-alkaline derivative, a liposome preparation including the derivative and use of the cabazitaxel weakly-alkaline derivative in a drug delivery system. The present invention belongs to the field of medical technology.

### BACKGROUND

Cabazitaxel (CTX) is a new generation of tubulin-binding agent of taxanes. Compared with paclitaxel and docetaxel, due to methylation of 7- and 10-hydroxyl groups, CTX has shown a low affinity for P-glycoprotein which closely associates with drug resistance in tumors, reduced efflux of drugs, and thus can better overcome the defect of multi-drug resistance. In June 2010, the U.S. Food and Drug Administration (FDA) has approved the use of cabazitaxel injections (trade name: Jevtana^{®}) produced by Sanofi-Aventis, France in combination with prednisone for treating hormone-resistant prostate cancer. However, Tween 80 in the preparation causes toxic and side effects associated with excipients, which severely limits the clinical application of the drug. In order to overcome the shortcomings of the cabazitaxel injection, it is necessary to redesign its core molecule and select a suitable preparation to increase the clinical application potential.

Liposomes have a phospholipid bilayer structure similar to that of a biological cell membrane, and may wrap drugs to isolate them from surrounding tissue, and reduce the irritation of the drugs by controlling the release of drugs. Compared with other preparations, a liposome preparation has good biocompatibility. By PEG-modifying the phospholipid, the liposome may be endowed with a long-circulation function, which can significantly prolong a circulation time of the drug in the body, thereby making the nano-sized liposome pass through a tumor blood vessel to reach a tumor site by means of enhanced permeability and retention effect (EPR effect) of tumors. Therefore, the tumors' targeting property is indirectly improved, the drug's uptake in the tumor site is increased, and the drug's release in normal tissues is reduced, thereby achieving the effects of "reducing toxicity and increasing efficacy".

At present, for most of liposome preparations used clinically, drugs are encapsulated through an active drug loading method. Compared with a passive drug loading method, the active drug loading method has the characteristics of high drug loading capacity, high encapsulation rate, and good stability of the preparation. In addition, as the drug is encapsulated in an internal aqueous phase of the liposome, the drug is prevented from leaking during a systemic circulation process. In the active drug loading method, it usually requires a more stable combination of the drug and a trapping agent in the internal aqueous phase, so that drug molecules present in the external aqueous phase enter the internal aqueous phase of the liposome through transmembrane driving force under certain conditions and stably exist. Cabazitaxel, as an electrically neutral compound, does not have weak acidity or weak basicity, thus it needs to be weakly alkalized so that it can be stably encapsulated in the internal aqueous phase of the liposome through the active drug loading method. Through the EPR effect, the nano-sized liposome preparation is targeted to the tumor site and then releases the encapsulated cabazitaxel derivative, which may release the active drug again after being hydrolyzed by esterase in vivo.

### SUMMARY

In order to overcome the shortcomings of existing cabazitaxel injections, the inventors of the present invention design a cabazitaxel weakly-alkaline derivative and prepares the derivative into a liposome of the derivative by encapsulating it, so as to obtain a nanoliposome preparation having a high drug loading capacity, a high encapsulation rate and good stability.

An object of the present invention is to weak-base modify cabazitaxel to prepare it into a nano-drug delivery system of liposomes, and endow it with long-circulation properties in blood for anti-tumor research.

The object of the present invention is achieved through the following technical solutions.

For a cabazitaxel weakly-alkaline derivative according to the present invention, cabazitaxel is connected to a weakly-alkaline intermediate through an ester bond, and the ester bond may be broken under the action of esterase in vivo so as to release an active drug. A structural formula of the cabazitaxel weakly-alkaline derivative is as follows: 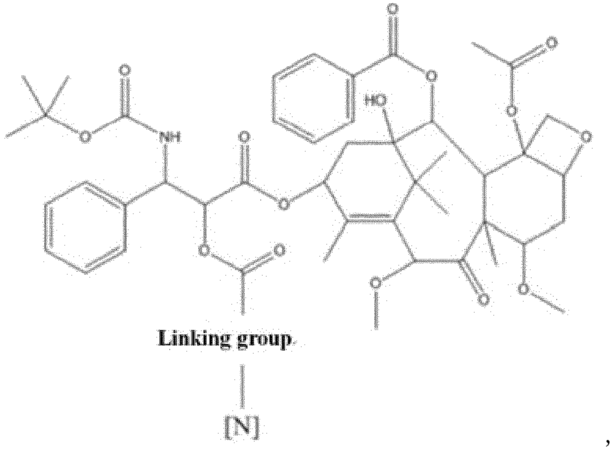 wherein
the linking group is C₁-C₄ alkyl, C₃-C₆ cycloalkyl or phenyl;
the [N] is N-methylpiperazinyl, piperidinyl, 4-(1-piperidinyl) piperidinyl, morpholinyl, tetrahydropyrrolyl or other tertiary amine structure.

Further, according to the present invention, a cabazitaxel weakly-alkaline derivative having the following structure is preferred:

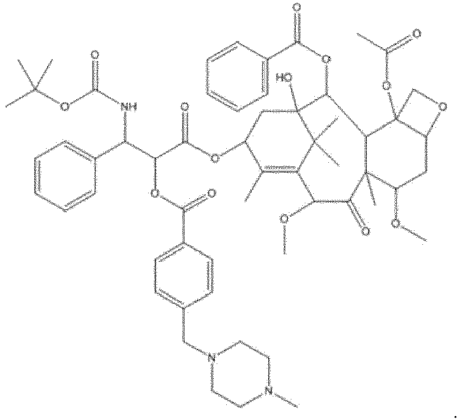

A synthesis method of the cabazitaxel weakly-alkaline derivative according to the present invention is as follows:
under the catalysis of DMAP, performing esterification reaction between 4-(4-methylpiperazinylmethyl) benzoyl chloride and cabazitaxel, and then performing separation and purification to obtain the cabazitaxel weakly-alkaline derivative, wherein the whole reaction process is performed under the protection of N₂, and DMAP may be replaced with triethylamine.

Further, the present invention provides a liposome including a cabazitaxel weakly-alkaline derivative, wherein the liposome includes the cabazitaxel weakly-alkaline derivative, phospholipid, cholesterol, PEGylated phospholipid, and the like. The weight ratio of the derivative to total lipids is 1: 4-12, and the total lipids is a sum of phospholipid, cholesterol and PEGylated phospholipid. The phospholipid, cholesterol and PEGylated phospholipid are used in conventional amounts in the art.

The present invention further provides a preparation method of a liposome of the cabazitaxel weakly-alkaline derivative, comprising step of:
(1) weighing a membrane material required for preparing the liposome, dissolving the membrane material into an organic solvent, and performing evaporation under reduced pressure to form a dry lipid membrane;
(2) adding an internal aqueous phase solution to the dry lipid membrane acquired in step (1), performing hydration at a temperature higher than a phase transition temperature, and sequentially squeezing a resulting product through polycarbonate membranes with different pore sizes to form nano-sized small unilamelar liposome;
(3) replacing an external aqueous phase of the small unilamelar liposome acquired in step (2) to acquire a blank liposome having a gradient between the internal aqueous phase and the external aqueous phase; and
(4) adding an organic solution of the cabazitaxel weakly-alkaline derivative to the blank liposomes having a gradient acquired in step (3), and performing incubation to acquire a liposome preparation of the cabazitaxel weakly-alkaline derivative.

Preferably, in step (2), the internal aqueous phase solution may be a citric acid solution, an ammonium sulfate solution, a sulfobutyl ether-β-cyclodextrin triethylammonium salt solution, or a sucrose octasulfate triethylammonium salt solution.

Further preferably, the internal aqueous phase solution is an ammonium sulfate solution.

Preferably, in step (3), the external aqueous phase solution may be a sucrose solution, HEPES buffer, phosphate buffer, or acetate buffer.

Further preferably, the external aqueous phase solution is a sucrose solution.

Preferably, in step (4), a solvent of the organic solution may be methanol, ethanol, acetone, tetrahydrofuran, acetonitrile, or DMSO.

Further preferably, a solvent of the organic solution is ethanol.

In the present invention, cabazitaxel is prepared into the weakly-alkaline derivative for preparing a liposome, which can not only avoid the side effects caused by Tween 80 in a cabazitaxel injection, but also increase the maximum tolerated dose of a drug. Thus, the anti-tumor effect of the drug is enhanced and great clinical application potential is achieved.

The liposome nano-drug delivery system according to the present invention has the advantages as follows: (1) the particle size is small and uniform (less than 100 nm), which is beneficial for enrichment of the drug to a tumor site through the EPR effect; (2) the drug loading capacity is high, which is beneficial for reduction of adverse reactions caused by excipients and biological materials; (3) complete drug encapsulation can be acquired, and good stability and ease industrialization are achieved; (4) the uptake by a reticuloendothelial system is effectively avoided, a long circulation effect is achieved in blood and the probability that the drug reaches the tumor site is increased; and (5) compared with commercially available preparations, the liposome nano-drug delivery system according to the present invention has an improved antitumor effect and reduced toxic and side effects.

### BRIEF DESCRIPTION OF THE DRAWINGS

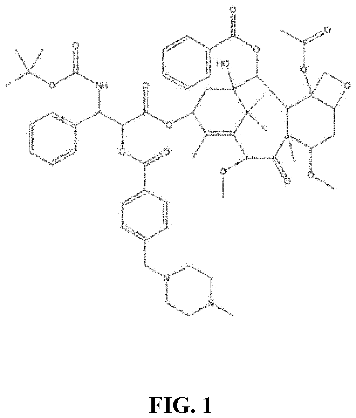

FIG. 1 is a diagram showing structure formula of a cabazitaxel derivative (CN1) having a basic group part of 4-(4-methylpiperazinemethyl) phenyl, according to Example 1 of the present invention.
FIG. 2 is a diagram showing ¹H-NMR spectrum of the cabazitaxel derivative (CN1) having a basic group part of 4-(4-methylpiperazinemethyl) phenyl, according to Example 1 of the present invention.

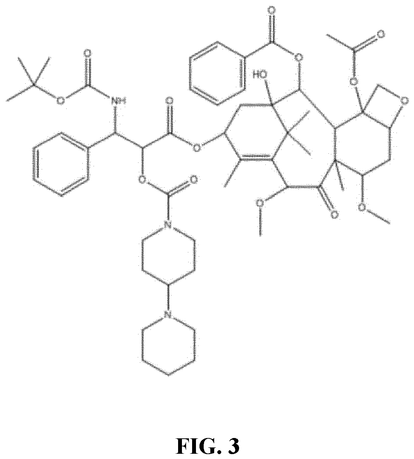

FIG. 3 is a diagram showing structure formula of a cabazitaxel derivative (CN2) having a basic group part of 4-(1-piperidinyl) piperidinyl, according to Example 2 of the present invention.
FIG. 4 is a diagram showing ¹H-NMR spectrum of the cabazitaxel derivative (CN2) having a basic group part of 4-(1-piperidinyl) piperidinyl, according to Example 2 of the present invention.

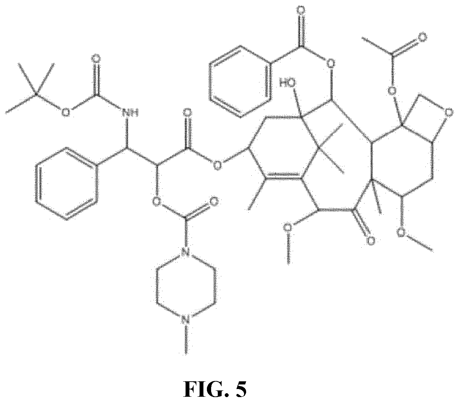

FIG. 5 is a diagram showing structure formula of a cabazitaxel derivative (CN3) having a basic group part of 4-methylpiperazine-1-methyl, according to Example 3 of the present invention.
FIG. 6 is a diagram showing ¹H-NMR spectrum of the cabazitaxel derivative (CN3) having a basic group part of 4-methylpiperazine-1-methyl, according to Example 3 of the present invention.
FIG. 7 is a diagram showing a particle size, an encapsulation ratio and a storage time of a liposome (CN1-liposome) of the cabazitaxel derivative according to Example 5 of the present invention.
FIG. 8 is a diagram showing a change in tumor volume in an in-vivo anti-tumor experiment using the liposome of the cabazitaxel derivative according to Example 7 of the present invention.
FIG. 9 is a diagram showing a change in body weight of a mouse in an in-vivo anti-tumor experiment using the liposome of the cabazitaxel derivative according to Example 7 of the present invention.

Table 1 is pharmacokinetic parameters of liposomes of cabazitaxel derivative (CN1-liposomes) according to Example 6 of the present invention.

### EMBODIMENTS

The following embodiments are intended to further illustrate the present invention without limiting the present invention in any way.

### Example 1: Synthesis of a cabazitaxel derivative (CN1) having a basic group part of 4-(4-methylpiperazinemethyl) phenyl

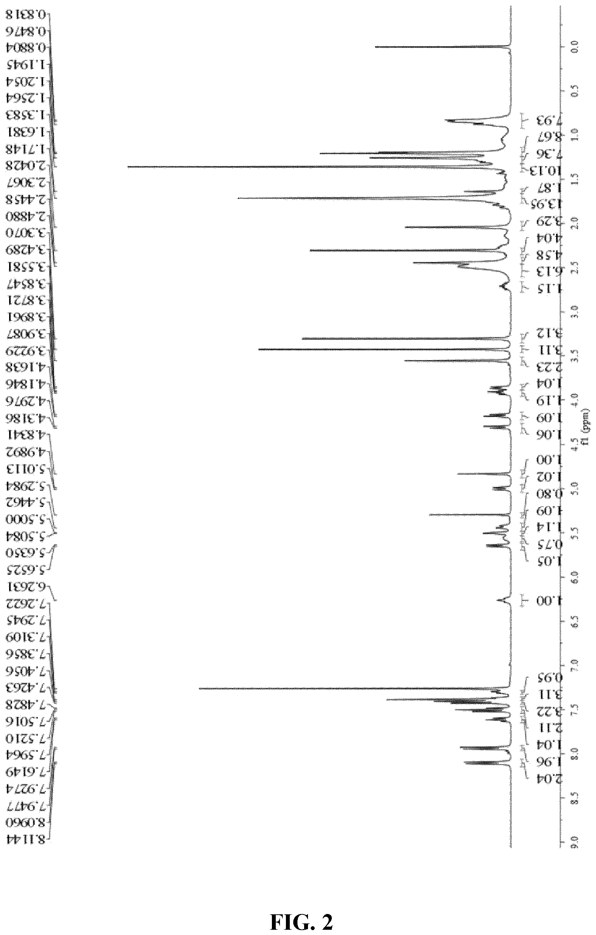

Cabazitaxel (200 mg, 0.24 mmol) and 4-(4-methylpiperazinylmethyl) benzoyl chloride (156 mg, 0.48 mmol) were weighed and dissolved in dichloromethane; 0.25 mL of triethylamine was added thereto; under an ice bath, a dichloromethane solution of DMAP (5.9 mg, 0.048 mmol) was slowly added dropwise to the thus obtained mixture; and stirring was performed at the room temperature overnight under N₂ protection. After the reaction was completed, separation and purification were performed through column chromatography to acquire a cabazitaxel derivative in the form of a white powder (yield: 95.01%). The structure of the compound in Example 1 was determined through a nuclear magnetic resonance hydrogen spectrum. The results are shown in FIG. 2: Spectral analysis results are as follows:
¹H NMR (Chloroform-d,400MHz) δ8.11 (2H,d,J=7.4Hz), 7.94 (2H,d,J=8.1 Hz), 7.61 (1H,t,J=7.4Hz), 7.51 (1H,d,J=7.8Hz), 7.48 (1H,d,J=7.8Hz), 7.45-7.35(6H,m), 7.32-7.27(1H,m), 6.26 (1H,t,J=9.1Hz), 5.64 (1H,d,J=7.0Hz), 5.50 (1H,d,J=3.4Hz), 5.43(1H,d,J=9.4Hz), 5.30 (1H,s), 5.00(1H,d,J=8.8Hz), 4.83 (1H,s), 4.31 (1H,d,J=8.4Hz), 4.17(1H,d,J=8.3Hz), 3.97-3.88(1H,m), 3.86 (1H,d,J=7.0Hz), 3.56(2H,s), 3.43(3H,s), 3.31(3H,s), 2.82-2.62(2H,m), 2.54- 2.38(6H,m), 2.31(3H,s), 2.25(1H,m), 2.04(3H,s), 1.82(1H,m), 1.79(1H,m), 1.71 (3H,s), 1.64 (2H,s), 1.36 (9H,s), 1.26(3H,s), 1.21(6H,s).

### Example 2: Synthesis of a cabazitaxel derivative (CN2) having a basic group part of 4-(1-piperidinyl) piperidinyl

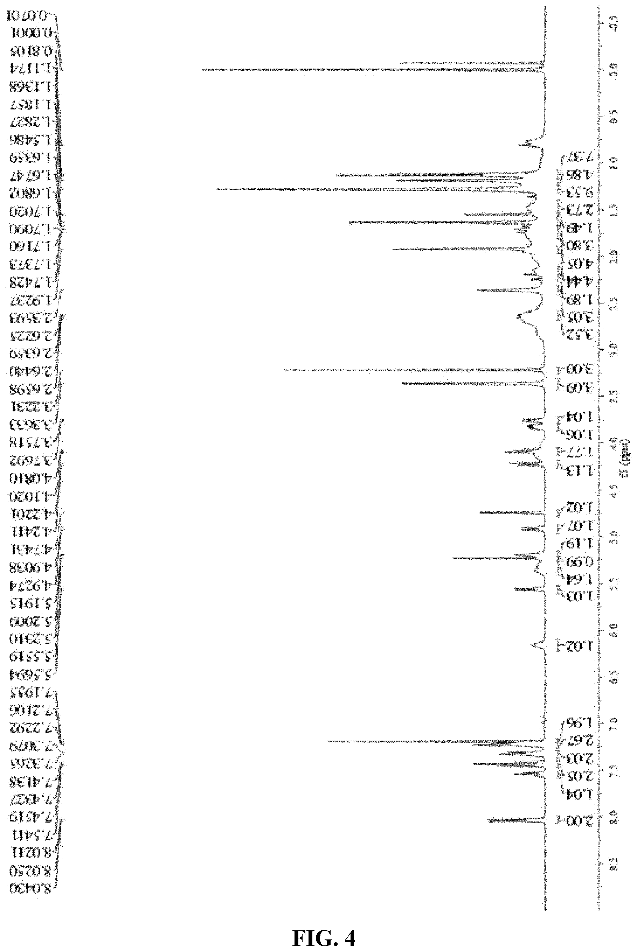

Cabazitaxel (200 mg, 0.24 mmol) and 4-piperidylpiperidinecarbonyl chloride (111mg, 0.48 mmol) were weighed and dissolved in dichloromethane; 0.25 mL of triethylamine was added thereto; under an ice bath, a dichloromethane solution of DMAP (5.9 mg, 0.048 mmol) was slowly added dropwise to the thus obtained mixture; and stirring was performed at the room temperature overnight under N₂ protection. After the reaction was completed, separation and purification were performed through column chromatography to acquire a cabazitaxel derivative in the form of a white powder (yield: 95%). The structure of the compound in Example 2 was determined through a nuclear magnetic resonance hydrogen spectrum. The results are shown in FIG. 4: Spectral analysis results are as follows:
¹H NMR (400MHz, Chloroform-d) δ8.07-8.00 (m,2H), 7.54(t,J=7.4Hz, 1H), 7.43 (t,J=7.6Hz,2H), 7.33 (t,J=7.6Hz,2H), 7.22 (m,3H), 6.32-5.95 (m, 1H), 5.56 (d,J=7.0Hz,1H), 5.44-5.26 (m,2H), 5.23 (s,1H), 5.20 (d,J=3.8Hz, 1H), 4.92 (d,J=9.4Hz,1H), 4.74(s,1H), 4.23 (d,J=8.4Hz,1H), 4.09 (d,J=8.4 Hz,2H), 3.83 (dd,J=10.7,6.4Hz,1H), 3.76 (d,J=7.0Hz,1H), 3.36(s,3H), 3.22(s, 3H), 2.77-2.49 (m,4H), 2.36 (s,3H), 2.27-2.05 (m,1H), 1.92(s,3H), 1.77-1.66 (m,1H), 1.64(s,3H), 1.55(s,1H), 1.52-1.39 (m,2H), 1.28(s,9H), 1.19(s,3H), 1.14 (s,3H), 1.12(s,3H).

### Example 3: Synthesis of a cabazitaxel derivative (CN3) having a basic group part of 4-methylpiperazine-1-methyl

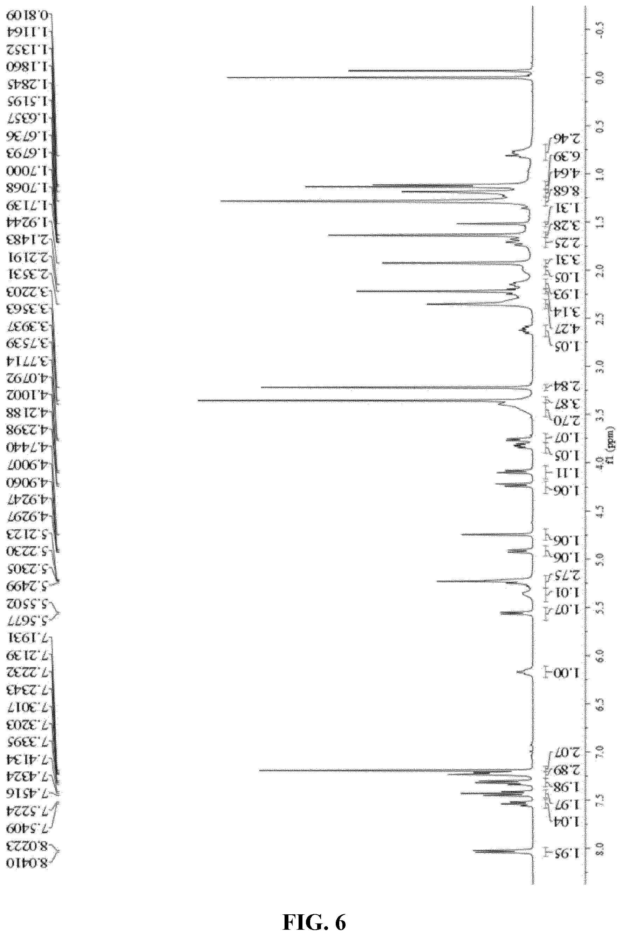

Cabazitaxel (200 mg, 0.24 mmol) and 4-methylpiperazine-1-carbonyl chloridel (78 mg, 0.48 mmol) were weighed and dissolved in dichloromethane; 0.25 mL of triethylamine was added thereto; under an ice bath, a dichloromethane solution of DMAP (5.9 mg, 0.048 mmol) was slowly added dropwise to the thus obtained mixture; and stirring was performed at the room temperature overnight under N₂ protection. After the reaction was completed, separation and purification were performed through column chromatography to acquire a cabazitaxel derivative in the form of a white powder (yield: 93.8%). The structure of the compound in Example 3 was determined through a nuclear magnetic resonance hydrogen spectrum. The results are shown in FIG. 6: Spectral analysis results are as follows:
¹H NMR (400MHz,Chloroform-d) δ8.03 (d,J=7.5Hz,2H), 7.54 (t,J=7.4Hz, 1H), 7.43 (t,J=7.6Hz,2H), 7.32 (t,J=7.6Hz,2H), 7.26-7.20 (m,3H), 6.17(t,J= 9.3Hz,1H), 5.56 (d,J=7.0Hz,1H), 5.36 (s,1H), 5.25(s,1H), 5.23(s,1H), 4.92(dd, J=9.5,2.0Hz,1H), 4.74(s,1H), 4.23 (d,J=8.4Hz,1H), 4.09 (d,J=8.4Hz,1H), 3.83 (dd,J=10.7,6.3Hz,1H), 3.76(d,J=7.0Hz,1H), 3.39 (s,2H), 3.36 (s,3H), 3.22 (s,3H), 2.62 (ddd,J=14.1,9.8,6.3Hz,1H), 2.36(m,1H), 2.35(s,3H), 2.22(s, 3H), 2.15 (t,J=7.6Hz,2H), 2.00 (m,1H), 1.92 (s,3H), 1.78-1.66 (m,2H), 1.64(s, 3H), 1.52(s,1H), 1.28 (s,9H), 1.19(s,3H), 1.14(s,3H), 1.12(s,3H).

### Example 4: Preparation of liposomes of cabazitaxel derivative

The preparation method of the liposomes of cabazitaxel derivative according to the present example included the following steps:
(1) preparation of blank liposomes: DSPC, cholesterol, DSPE-mPEG₂₀₀₀ (a mass ratio of 3 : 1: 0.05) were weighted; chloroform was added to the above materials for dissolution; evaporation under reduced pressure was performed at 37 °C for removing an organic solvent to form a dry lipid membrane; 350mM ammonium sulfate solution was added for hydration at 65 °C for 30 minutes; and whole particles were filtered through a polycarbonate membrane to form small unilamelar liposomes having both of an internal aqueous phases and an external aqueous phases being the ammonium sulfate solution;
(2) preparation of gradient blank liposomes: the blank liposomes acquired in step (1) passed through an agarose CL-4B gel column previously equilibrated with 300 mM sucrose to acquire an ammonium ion gradient blank liposome having an internal aqueous phase of ammonium sulfate solution and an external aqueous phase of sucrose solution;
(3) drug loading process: an ethanol solution of a cabazitaxel derivative was added to the ammonium ion gradient blank liposomes prepared in step (2); and incubation was performed for 20 min at 60 °C to finally acquire the liposomes of cabazitaxel derivative (CN1-liposomes, CN2-liposomes and CN3-liposomes).

### Example 5: Colloidal stability test of liposomes of cabazitaxel derivative (CN1-lposomes)

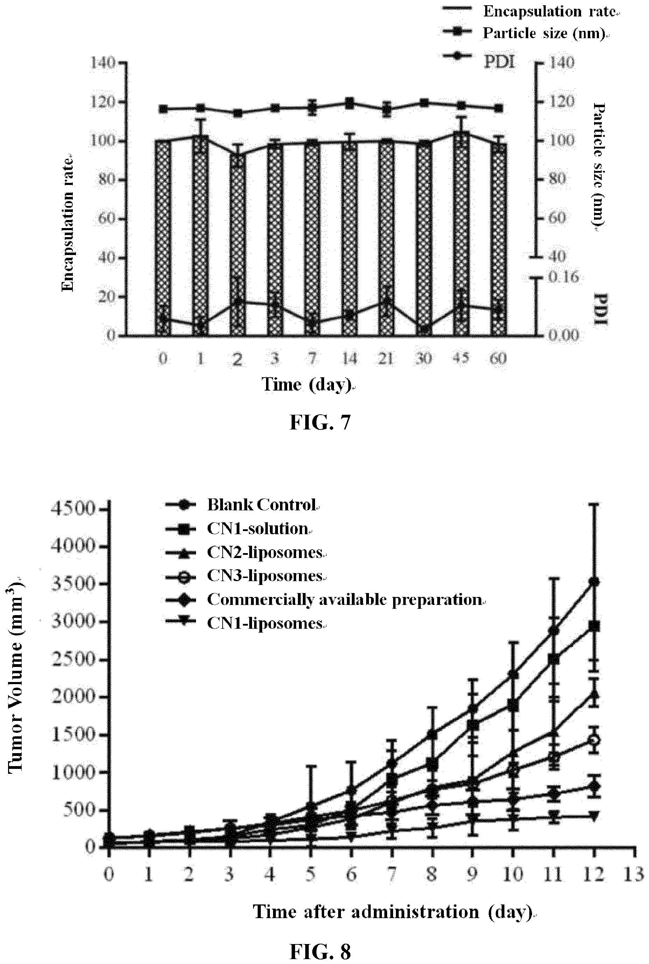

The liposomes preparation prepared in Example 4 was subjected to sterile filtration and stored at 4 °C for 60 days. During this period, a change in particle size was measured through a dynamic light scattering method, and a change in encapsulation rate was measured through high-performance liquid chromatography. The results were shown in FIG. 7: the particle size and the encapsulation rate of the actively drug-loaded liposomes of cabazitaxel derivative were not significantly changed within 60 days, thus they exhibiting good long-term storage stability. Example 6: pharmacokinetic study of liposomes of cabazitaxel derivative (CN1-liposomes) 10 healthy male rats with a weight of 200-250 g for each one, were randomly divided into 2 groups with 5 rats in each group, and these two groups of rats were injected with a commercially available preparation and the CN1-liposomes prepared in Example 4 at tail veins respectively, wherein the equivalent dosage of cabazitaxel was 5 mg/kg. Blood was taken from the orbits at a prescribed time and centrifuged to acquire plasma, and the concentration of a drug in the plasma was measured through high-performance liquid chromatography-mass spectrometry.

The results are shown in table 1. A circulation time of the liposomes preparation in the body was significantly prolonged, and the area under the concentration-time curve (AUC) of the liposome preparation was significantly improved. The experimental results showed that the liposome preparation can significantly prolong the circulation time of the drug in the blood, and increases the possibility that the drug accumulates to the tumor site through the EPR effect.

**Table 1: Pharmacokinetic parameters of liposomes of cabazitaxel derivative (CN1-liposomes)**

| | Commercially available preparation | CN1-liposomes |
|---|---|---|
| AUC_{(0-∞)} (ug/L^{∗}h) | 1342.167 ± 231.161 | 1277826.64 ± 433820.141 |
| MRT_{(0-∞)} (h) | 2.845 ± 0.814 | 6.207 ± 0.946 |
| t_{1/2} (h) | 3.393 ± 1.562 | 4.497 ± 0.732 |
| CLz (L/h/kg) | 3.816 ± 0.667 | 0.005 ± 0.002 |
| Cmax (ug/L) | 1259.42 ± 227.789 | 270060.434 ± 142270.937 |

### Example 7: Animal pharmacodynamics experiment of liposomes of cabazitaxel derivative

Mouse prostate cancer cells (RM-1, 5 ^{∗} 10⁶ cells/100 µL PBS) were inoculated subcutaneously on the right ventral sides of male C57BL/6 mice. After the tumors grew to 60-80 mm³, the mice were randomly divided into 6 groups with 5 mice in each group: a blank control group, a CN1-solution group, a commercially available preparation group, a CN1-liposomes group, a CN2-liposomes group, and a CN3-liposomes group. The drugs were administered every three days for a total of 5 times, wherein the equivalent dosage of cabazitaxel was 6 mg/kg, and the high dosage was three times of the low dosage. After administration, the states of the mice were observed, the mice were weighed and the volumes of the tumors were measured. At the end of the last dosing cycle, the mice were killed, tumors and major organs were stripped for analysis and evaluation.

The results are shown in FIG. 8, which shows that the CN1-solution group has almost no antitumor activity, whereas the CN1-liposomes group exhibits better antitumor activity than the commercially available preparation group. As a result, although the alkalized CN2 and CN3 may be successfully prepared into liposomes preparations, the antitumor activity of the CN2-liposomes group and the CN3-liposomes group is weaker than that of the CN1-liposomes group, and is also weaker than that of the commercially available preparation group.

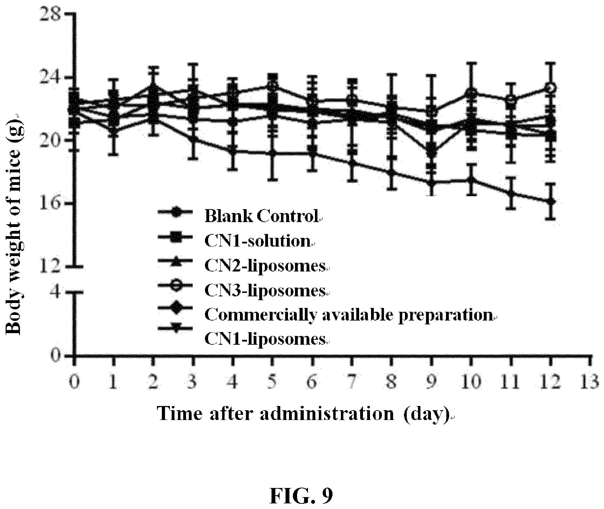

As shown in FIG. 9, the body weight of the mice in the commercially available preparation group is decreased significantly, indicating that the preparation has certain toxicity to the mice, whereas no significant body weight change is observed in the liposome preparation group. In summary, the CN1-liposomes group also has better anti-tumor activity and exhibits the effects of "reducing toxicity and increasing efficacy", and thus is a safe and effective anti-tumor drug delivery system and has good clinical application potential.

The above description only illustrates preferred embodiments of the present invention. It should be noted that those ordinary skilled person in the art can make several improvements and modifications without departing from the principles of the present invention, and these improvements and modifications shall fall into the protection scope of the present invention.

## Claims

1. A cabazitaxel weakly-alkaline derivative or a pharmaceutically acceptable salt thereof, wherein the cabazitaxel weakly-alkaline derivative has a structural formula as follows: 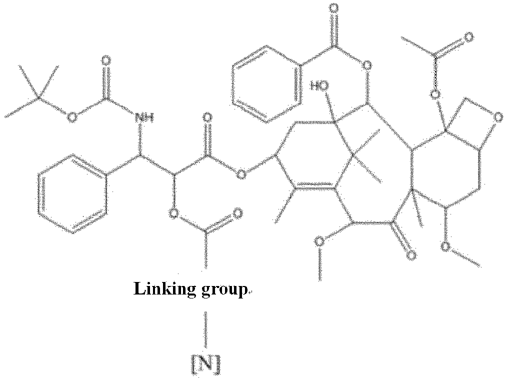 ,
where the linking group is C₁-C₄ alkyl, C₃-C₆ cycloalkyl or phenyl; and
the [N] is N-methylpiperazinyl, piperidinyl, 4-(1-piperidinyl) piperidinyl, morpholinyl, tetrahydropyrrolyl or other tertiary amine structure.

2. The cabazitaxel weakly-alkaline derivative or pharmaceutically acceptable salt thereof according to claim 1, wherein the cabazitaxel weakly-alkaline derivative has a structural formula as follows:

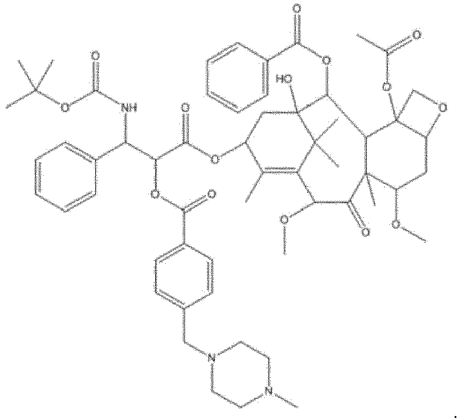

3. The cabazitaxel weakly-alkaline derivative or pharmaceutically acceptable salt thereof according to claim 1 or claim 2, wherein the pharmaceutically acceptable salt is a salt formed by the cabazitaxel weakly-alkaline derivative and a pharmaceutically acceptable inorganic or organic acid.

4. A method for synthesizing the cabazitaxel weakly-alkaline derivative according to claim 2, wherein the method comprises steps of: under the catalysis of DMAP, performing esterification reaction between 4-(4-methylpiperazinylmethyl) benzoyl chloride and cabazitaxel, and then performing separation and purification to obtain the cabazitaxel weakly-alkaline derivative, wherein the whole reaction process is performed under the protection of N₂, and DMAP is optionally replaced with triethylamine.

5. A liposome of cabazitaxel weakly-alkaline derivative, wherein the cabazitaxel weakly-alkaline derivative according to claim 2 is prepared into liposome; the liposome comprises the cabazitaxel weakly-alkaline derivative, phospholipid, cholesterol, and PEGylated phospholipid; and the liposome is prepared by steps of:
(1) preparing a blank liposome having a gradient;
(2) preparing an ethanol solution of the cabazitaxel weakly-alkaline derivative; and
(3) incubating the ethanol solution of the cabazitaxel weakly-alkaline derivative and the blank liposome having a gradient.

6. The liposome of cabazitaxel weakly-alkaline derivative according to claim 5, wherein
(1) weighing a membrane material required for preparing the liposome, dissolving the membrane material into an organic solvent, and performing evaporation under reduced pressure to form a dry lipid membrane;
(2) adding an internal aqueous phase solution to the dry lipid membrane acquired in step (1), performing hydration at a temperature higher than a phase transition temperature, and sequentially squeezing a resulting product through polycarbonate membranes with different pore sizes to form nano-sized small unilamelar liposome;
(3) replacing an external aqueous phase of the small unilamelar liposome acquired in step (2) to acquire a blank liposome having a gradient between the internal aqueous phase and the external aqueous phase; and
(4) adding an organic solution of the cabazitaxel weakly-alkaline derivative to the blank liposome having a gradient acquired in step (3), and performing incubation to acquire liposome preparation of cabazitaxel weakly-alkaline derivative, wherein the organic solvent may be removed by tangential ultrafiltration, dialysis, and the like.

7. The liposome of cabazitaxel weakly-alkaline derivative according to claim 6, wherein in step (2), the internal aqueous phase solution may be a citric acid solution, an ammonium sulfate solution, a sulfobutyl ether-β-cyclodextrin triethylammonium salt solution, a sucrose octasulfate triethylammonium salt solution or the like.

8. The liposome of cabazitaxel weakly-alkaline derivative according to claim 5, wherein a weight ratio of the cabazitaxel weakly-alkaline derivative to total lipids is 1: 4-12, and the total lipids is a sum of phospholipid, cholesterol and PEGylated phospholipid.

9. Use of the cabazitaxel weakly-alkaline derivative or a pharmaceutically acceptable salt thereof according to any one of claims 1 to 3 or the liposome of cabazitaxel weakly-alkaline derivative according to any one of claims 5 to 8 in preparation of a drug delivery system.

10. Use of the cabazitaxel weakly-alkaline derivative or a pharmaceutically acceptable salt thereof according to any one of claims 1 to 3 or the liposome of cabazitaxel weakly-alkaline derivative according to any one of claims 5 to 8 in preparation of anti-tumor drugs.
